# EUROPEAN PATENT SPECIFICATION

(11) **EP 4 408 362 B1**
(45) Date of publication and mention of the grant of the patent: **21.10.2026**
(21) Application number: 22877202.6
(22) Date of filing: 27.09.2022
(51) Int. Cl.: A61F 11/20, A61M 31/00, A61B 1/227

(54) **COCHLEA INJECTION SYSTEM**
COCHLEAINJEKTIONSSYSTEM
SYSTÈMES D'INJECTION COCHLÉAIRE

(30) Priority: 29.09.2021 US 202163249938 P
(43) Date of publication of application: 07.08.2024
(73) Proprietor: Spiral Therapeutics Inc., South San Francisco, CA 94080 (US)
(72) Inventor: ERICKSON, Signe, San Francisco, California 94080 (US); LEVERING, Vrad, San Francisco, California 94080 (US); DE JUAN, Eugene, San Francisco, California 94080 (US); AYOOB, Andrew, San Francisco, California 94080 (US)
(74) Representative: Fish & Richardson P.C.
(86) International application number: PCT/US2022/044846
(87) International publication number: WO 2023/055719

(56) References cited:
- WO-A1-2020/115674
- WO-A1-2020/128610
- US-A1- 2008 199 400
- US-A1- 2011 224 629
- US-A1- 2013 085 476
- US-A1- 2015 265 824
- US-A1- 2019 038 469
- US-A1- 2021 154 452
- US-A1- 2021 228 237

## Description

### CROSS-REFERENCE TO RELATED APPLICATION

This application claims the benefit of United States Provisional Application No. 63/249,938, filed September 29, 2021.

### TECHNICAL FIELD

This document relates to devices, systems, and methods for facilitating therapeutic procedures in the inner ear in order to treat otic disorders including, but not limited to, hearing loss and other ear disorders. In some examples, the systems and methods include instruments and techniques that can be used to minimize the invasiveness of procedures performed in the inner ear.

### BACKGROUND

Sensorineural Hearing Loss (SNHL) is due to the absence of, or damage to, hair cells in the cochlea, or to impairment of downstream neural signaling. SNHL is typically associated with exposure to loud noise, head trauma, aging, infection, Meniere's Disease, tumors, ototoxicity, genetic diseases like Usher Syndrome, and the like.

SNHL is common, and its impact on human communication and quality of life is significant. Consequences of SNHL range from moderate communication difficulty and social withdrawal to profound deafness and its significant challenges. Conventional management of SNHL typically involves the use of hearing aids or cochlear implants.

Effective administration of therapeutic agents for the treatment of SNHL is limited by several anatomical barriers. Systemically administered agents have difficulty crossing the blood labyrinth barrier and may require prohibitively high systemic doses to achieve a therapeutic level in the inner ear. Intratympanic administration can result in therapeutic exposure from diffusion across the round window and oval window membranes of the cochlea. However, this approach may result in a concentration gradient of therapeutic agents, with deeper regions of the cochlea remaining untreated. Direct administration to the round window membrane is desirable under certain circumstances, but it currently requires highly invasive surgical procedures that are costly and can lead to complications. Hence a safe, effective and minimally-invasive means to directly administer agents into the cochlea could transform the field of otology, enabling medical management of a range of inner ear disorders.

US 2013/085476 A1 describes systems and methods for delivering therapeutic compositions to the middle ear to treat inner ear disorders. An example of such a system comprises a sheath for insertion into the auditory canal and a cannula which can be inserted through the sheath. The cannula can include a tip for piercing the tympanic membrane and delivering the composition to a middle ear tissue surface. The system may also include a therapeutic composition comprising a therapeutic agent and a thixotropic material which allows the composition to be delivered (e.g., by injection) through the cannula in liquid form and transition to gel-form once delivered to the tissue surface. A method for using the system comprises introducing the sheath into the ear, introducing the cannula through the sheath to pierce the tympanic membrane and position the tip adjacent a middle ear tissue surface; and delivering the composition through the cannula to adhere to the tissue surface.

WO 2020/128610 A1 describes a device for visualizing and providing suction for a surgical procedure in an ear may include a handle, a main shaft extending from the handle, an imaging sensor at a distal end of the main shaft, a light source at the distal end of the main shaft, and a suction shaft extending from the handle. The device may also include a spring coupled with the suction shaft and/or the handle, such that when the suction shaft is advanced in a distal direction and then released, the suction shaft retracts automatically. The suction shaft may have a distal curved portion, and the device may have a thumb depress portion that allows a user to spin the suction shaft to suction in different directions. Some embodiments may include a suction shaft with a sharp distal tip that is configured for placing an ear tube in the tympanic membrane.

### SUMMARY

According to the invention, there is provided a system for injecting a therapeutic agent into the cochlea according to claim 1 and the dependent claims.

This document describes devices, systems, and methods for facilitating administration of therapeutic agents into the inner ear (cochlea) in order to treat a range of inner ear disorders and forms of SNHL. These include idiopathic and inflammatory conditions affecting the inner ear, including autoimmune inner ear disease, chemotherapy induced ototoxicity, and Meniere's disease. In addition, other degenerative inner ear disorders may be amenable to treatment with such a system including idiopathic, genetically-based, and age-related progressive SNHL. In some examples, the systems and methods include instruments and techniques that can be used to minimize the invasiveness of drug delivery or surgical procedures performed in the inner ear.

The devices, systems, and methods described herein can be used in conjunction with additional treatment techniques. For example, the devices, systems, and methods described herein can be used in conjunction with treatment techniques such as, but not limited to, therapeutic agent delivery (which can be in the form of a liquid, gel, solid, etc.) including, small molecules, proteins, and gene therapy vectors, device or implant delivery, diagnostic procedures, and surgical procedures, among others.

A system according to the present invention is defined in claim 1. Further advantageous developments of the present invention are set out in the dependent claims.

In one aspect, this disclosure is directed to a method for injecting a therapeutic agent into a cochlea of a patient. In some embodiments, such a method includes advancing, via an outer ear canal of the patient, a shaft of an endoscope such that a distal tip of the endoscope is positioned in a middle ear region of the patient; advancing, through a working channel of the endoscope, a micro-cannula defining a lumen; advancing, through the lumen of the micro-cannula, a first guidewire; piercing a round window membrane of the patient using a tip of the first guidewire; and injecting, via the lumen of the micro-cannula, the therapeutic agent into the cochlea.

Such a method for injecting a therapeutic agent into a cochlea of a patient may optionally include one or more of the following features. The first guidewire may have an end portion with a curved shape. The method may also include advancing the micro-cannula over the end portion of the first guidewire, wherein, as the micro-cannula is advanced over the end portion of the first guidewire, the micro-cannula assumes the curved shape and becomes aligned toward the round window membrane. The method may also include, after piercing the round window membrane and prior to the injecting, advancing the micro-cannula within a scala tympani of the cochlea. Advancing the micro-cannula within the scala tympani may be performed without advancing the first guidewire. Advancing the micro-cannula within the scala tympani may be performed while also advancing the first guidewire. Advancing the micro-cannula within the scala tympani may be performed after advancing the first guidewire within the scala tympani. The method may also include, after piercing the round window membrane and prior to the injecting, withdrawing the first guidewire from the lumen of the micro-cannula and inserting a second guidewire into the lumen of the micro-cannula. The method may also include, after piercing the round window membrane and prior to the injecting, advancing the second guidewire within a scala tympani of the cochlea. The method may also include, after advancing the second guidewire within the scala tympani of the cochlea, advancing the micro-cannula over the second guidewire and into the scala tympani of the cochlea. In some embodiments, the injecting the therapeutic agent into the cochlea is performed while the first guidewire is in the lumen of the micro-cannula. The method may also include withdrawing the first guidewire from the lumen of the micro-cannula, wherein the injecting the therapeutic agent into the cochlea is performed after the first guidewire is withdrawn from the lumen of the micro-cannula. The method may also include aspirating fluid from the cochlea while performing the injecting the therapeutic agent into the cochlea. The aspirating fluid from the cochlea may be modulated based on a fluid pressure measured in the cochlea. The aspirating fluid from the cochlea may be modulated based on a volumetric balance between the therapeutic agent injected and the fluid aspirated. The micro-cannula may include depth markers. The method may also include, after piercing the round window membrane and prior to the injecting, advancing the micro-cannula within a scala tympani of the cochlea and using the depth markers to determine a depth to which a tip of the micro-cannula is advanced within the scala tympani. The method may also include creating an incision in a tympanic membrane of the patient, and wherein the shaft of the endoscope is advanced through the incision.

In another aspect, this disclosure is directed to a method for injecting a therapeutic agent into a cochlea of a patient. The method includes: advancing, via an outer ear canal of the patient, a shaft of an endoscope such that a distal tip of the endoscope is positioned in a middle ear region of the patient; advancing, through a working channel of the endoscope, a micro-cannula defining a lumen, wherein a distal tip of the micro-cannula is advanced through a round window membrane and to a depth within a scala tympani of the cochlea; injecting, via the lumen of the micro-cannula and while the distal tip of the micro-cannula is at the depth within the scala tympani, the therapeutic agent into the cochlea; and during the injecting, aspirating fluid from the scala tympani.

Such a method for injecting a therapeutic agent into a cochlea of a patient may optionally include one or more of the following features. The aspirating may be performed using a second lumen of the micro-cannula. The aspirating may be modulated based on a fluid pressure measured in the cochlea. The aspirating may be modulated based on a volumetric balance between the therapeutic agent injected and the fluid aspirated.

In another aspect, this disclosure is directed to method for injecting a therapeutic agent into a cochlea of a patient. The method includes: injecting the therapeutic agent via a micro-cannula and while a distal tip of the micro-cannula is at a depth within a scala tympani of the cochlea; and during the injecting, aspirating fluid from the scala tympani.

In another aspect, this disclosure is directed to a method for injecting a therapeutic agent into a cochlea of a patient. The method includes: advancing a guidewire within a scala tympani of the cochlea; advancing a micro-cannula over the guidewire within the scala tympani to position a distal tip of the micro-cannula at a depth within the scala tympani; and injecting, via a lumen of the micro-cannula, the therapeutic agent into the cochlea.

In another aspect, this disclosure is directed to a medical device system that includes: an endoscope with a distal shaft sized and configured to be positioned in a middle ear via an incision in a tympanic membrane, the endoscope defining a working channel; and a micro-cannula slidably disposable within the working channel and defining a lumen.

Such a medical device system may optionally include one or more of the following features. The medical device system may also include a guidewire slidably disposable within the lumen. The guidewire may have a curved distal end portion. The micro-cannula may have a lateral compliance such that a portion of the micro-cannula becomes curved when the curved distal end portion of the guidewire is engaged within the lumen alone the portion of the micro-cannula.

Some or all of the embodiments described herein may provide one or more of the following advantages. First, some embodiments of instruments and associated techniques for treating inner ear disorders as described herein reduce the procedural invasiveness in comparison to conventional methods. For example, instruments and techniques are disclosed for accessing inside the cochlea using a trans-canal and trans-tympanic membrane approach (and other approaches). Moreover, the trans-tympanic membrane approach can be via a small, self-healing incision in the tympanic membrane. Such minimally invasive procedures can reduce overall procedural time and costs, and mitigate risks to patients that are associated with more invasive procedures.

Second, in some embodiments the use of the instruments and techniques described herein can treat conditions of the inner ear without perforation of the oval window or creation of a second fenestration of the cochlea. For example, in some procedures described herein a therapeutic agent can be infused into the cochlea via the round window without any effect to the oval window. Such techniques can advantageously serve to maintain better control of the injectate as compared to techniques that include a perforation of the oval window. Such techniques also reduce risks to patients associated with creation of a fistula.

Third, in some embodiments aspiration of cochlear fluid can be performed concurrently with an infusion of a therapeutic agent into the cochlea. In some such embodiments, a fluid balance within the cochlea can be achieved or approximated. Accordingly, pressure changes within the cochlea associated with an infusion of a therapeutic agent can be advantageously eliminated or closely controlled. Such aspiration can be active or passive. Cochlear fluid which is collected during the procedure can be analyzed for biomarkers which can aid in the diagnosis of disease.

Fourth, the devices, systems, and methods described herein advantageously allow the treatment of otic conditions within the cochlea without affecting the chorda tympani nerve. In contrast, some conventional methods that include an infusion of a therapeutic agent into the cochlea to treat otic conditions might require damaging (e.g., cutting) the chorda tympani nerve. The techniques described herein do not incur damage to the chorda tympani nerve.

Fifth, the techniques described herein do not rely on diffusion of a therapeutic agent through the round window membrane, or diffusion of a therapeutic agent within the cochlea. Instead, the techniques described herein enable a convective infusion of a therapeutic agent to targeted locations deep inside the cochlea and to an extent that results in desired therapeutic effects without relying on diffusion.

Sixth, the systems described herein can advantageously utilize the properties of one or more guidewires for various purposes such as, but not limited to, steering of a micro-cannula, adding column strength to a micro-cannula, tissue penetration or manipulation, and so on.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a perspective view of an example otic endoscope system in accordance with some embodiments.
FIG. 2 is a side view of a distal end portion of the otic endoscope system of FIG. 1, including an example micro-cannula.
FIG. 3 is another side view of the distal end portion of the otic endoscope system of FIG. 1, including an example guidewire extending from within the micro-cannula.
FIG. 4 is another side view of the distal end portion of the otic endoscope system of FIG. 1, with the micro-cannula advanced over the guidewire to cause a curve of the micro-cannula in accordance with the shape of the guidewire.
FIG. 5 illustrates the otic endoscope system of FIG. 1 being used to access a round window niche of an inner ear in accordance with some embodiments.
FIG. 6 illustrates a trans-tympanic incision through which the distal end portion of the otic endoscope system of FIG. 1 can extend to access the inner ear.
FIG. 7 illustrates an example cochlea with its tortuous anatomical pathway from the round window to inner regions of the cochlea.
FIGs. 8A and 8B provide a flowchart of an example method for infusing a therapeutic agent into a cochlea in accordance with some embodiments.

Like reference symbols in the various drawings indicate like elements.

### DETAILED DESCRIPTION OF ILLUSTRATIVE EMBODIMENTS

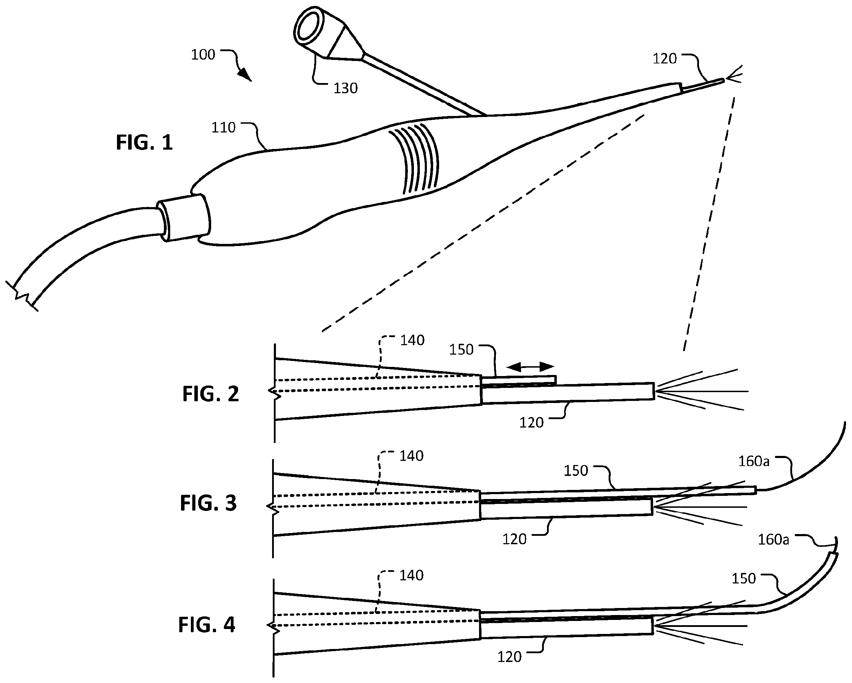

Referring to **FIG. 1****,** an example otic endoscope system 100 can be used to treat various otic conditions by accessing the inside of a cochlea, as described further below. The endoscope system 100 can broadly include a handle 110, an endoscope shaft 120, and a working channel access port 130. While the depicted embodiment includes a single working channel access port 130, in some embodiments two or more of the working channel access ports 130 can be included (along with two or more working channels associated with the working channel access ports 130).

The endoscope shaft 120 extends distally from the handle 110. In the depicted embodiment, the endoscope shaft 120 extends essentially parallel to the axis of the handle 110. However, in some embodiments the endoscope shaft 120 extends at a non-zero angle relative to the axis of the handle 110. For example, in some embodiments the endoscope shaft 120 extends at an angle between 0° and 45° relative to the handle 110.

In some embodiments, the endoscope shaft 120 emits visible light that can illuminate the middle ear and/or inner ear during use. In some embodiments, the light can originate from an external source and be transmitted to the distal tip of the endoscope shaft 120 via one or more fiber optics. Alternatively or additionally, in some embodiments the light can originate from an on-board source (e.g., an LED) mounted at or near the distal tip of the endoscope shaft 120. In some embodiments, light to illuminate the middle ear and/or inner ear can originate from a source (e.g., fiber optic, LED, etc.) that is located proximally of the distal tip of the endoscope shaft 120.

In the depicted embodiment, the otic endoscope system 100 is designed to be used with an external video display. Alternatively, in some embodiments the otic endoscope system 100 can include an integrated lens viewing system for directly observing the field of view of the endoscope shaft 120.

The otic endoscope system 100 also includes the working channel access port 130. The working channel access port 130 is an opening to a working channel that can receive one or more devices or instruments, as described further below.

Referring also to FIGs. 2-4, a working channel 140 can be accessed via the working channel access port 130. In this example, the working channel 140 is arranged to direct a device or instrument to extend parallel to endoscope shaft 120.

In the depicted embodiment, an example micro-cannula 150 is slidably disposed within the working channel 140. Accordingly, the micro-cannula 150 can be extended and retracted relative to the endoscope shaft 120 (as indicated by the double arrowed line in FIG. 2).

In addition, in this example the otic endoscope system 100 includes an example guidewire 160a (which can instead be a stylet and/or another micro-cannula in some embodiments). The guidewire 160a is slidably disposed within a lumen defined by the micro-cannula 150. Said another way, the micro-cannula 150 is over the guidewire 160a. Accordingly, guidewire 160a can be extended and retracted relative to the micro-cannula 150. In addition, the micro-cannula 150 can be extended and retracted relative to the guidewire 160a. These movements of the micro-cannula 150 and/or guidewire 160a can be manually actuated by a clinician in accordance with known techniques.

As described further below, various different guidewires can be utilized in conjunction with the otic endoscope system 100. In some cases, two or more different guidewires can be used during a single procedure. Such different guidewires can have different properties (e.g., shapes, stiffnesses, diameters, etc.) that can be put to use for particular purposes during particular stages of the therapeutic procedures described herein. Accordingly, the guidewire 160a is just one example of the types of guidewires that can be useful for the therapeutic procedures described herein.

As illustrated by FIGs. 3 and 4, the example guidewire 160a can have, but not need have, a natural curved end portion. In the depicted embodiment, the lateral stiffness of the curved end portion of the guidewire 160a is greater than the lateral stiffness of the micro-cannula 150. Said another way, the micro-cannula 150 is more compliant than the curved end portion of the guidewire 160a. Accordingly, when the micro-cannula 150 is distally extended over the curved end portion of the guidewire 160a the micro-cannula 150 takes on the curved shape of the guidewire 160a. In this manner, the micro-cannula 150 is deflected or steered as the micro-cannula 150 is advanced. In another embodiment, the guidewire 160a does not necessarily have a greater stiffness than the micro-cannula 150 when extended in air. However, once the guidewire 160a is advanced (for example, advanced into a round window niche requiring a curve in the guidewire 160a as described further below) where a portion of the guidewire 160a is braced, then the micro-cannula 150 tracks along the path of the guidewire 160a. If the guidewire 160a has been used to make a turn, then the micro-cannula 150 tracks along said path of the guidewire 160a.

In another embodiment, the guidewire 160a can be a smaller, inner micro-cannula sized to fit within the micro-cannula 150, and can itself have a stylet or guidewire riding inside its lumen. In some embodiments, this inner micro-cannula 160a can be constructed from Nitinol and have pre-shaped curve similar to the guidewire 160a. In some cases such a Nitinol inner micro-cannula (instead of the guidewire 160a) can be advantageous due to being able to maintain a more angulated curve than the guidewire 160a, thereby allowing for use in situations with tighter turns and/or greater turns.

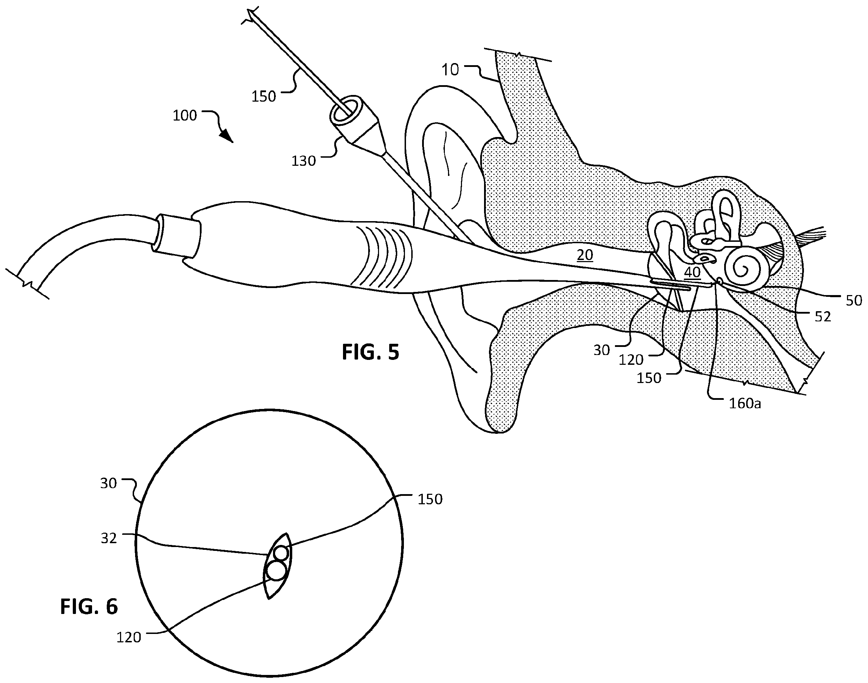

Referring to **FIG. 5****,** the otic endoscope system 100 can be used to treat a cochlea 50. The inside of the cochlea 50 (e.g., scala tympani) can be accessed by the guidewire 160a and/or the micro-cannula 150 of the otic endoscope system 100 via a round window 52 of the cochlea 50. In some embodiments, an infusion of a therapeutic agent can be delivered to within the cochlea 50 (e.g., scala tympani) via the micro-cannula 150, as described further below.

As shown, the otic endoscope system 100 can be extended into the outer ear 20 of a patient 10. Then, the micro-cannula 150 and the endoscope shaft 120 at the distal end portion of the otic endoscope system 100 can be advanced through a small (e.g., < 3mm) incision 32 (see **FIG. 6****)** in the tympanic membrane 30, and then extended into the middle ear 40. In this orientation, a clinician can manipulate the micro-cannula 150 and/or guidewire 160a within the middle ear 40 while using the endoscope shaft 120 to view the movements of the micro-cannula 150 and/or guidewire 160a resulting from the manipulations.

For instance, in the depicted example the guidewire 160a is being extended to enter the niche of the round window 52. This is done with visibility by the clinician using the endoscope shaft 120 positioned in the middle ear 40. In addition, with the guidewire 160a within the niche of the round window 52, then the clinician can extend the micro-cannula 150 over the guidewire 160a and into the niche of the round window 52.

In some cases, the patient 10 may have pseudomembrane over the niche of the round window 52. In such a case, the guidewire 160a or another instrument may be used to puncture and/or remove the pseudomembrane (or a portion thereof) prior to extending the guidewire 160a and/or micro-cannula 150 into the niche of the round window 52.

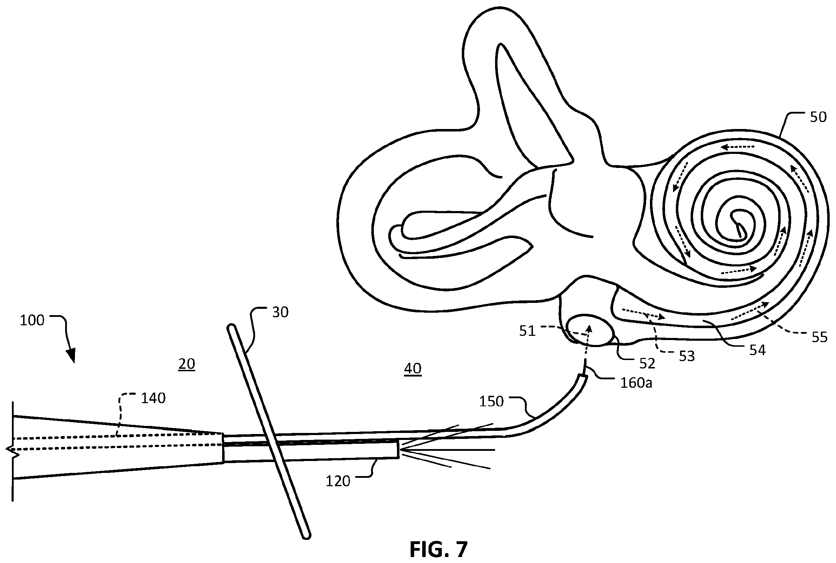

**FIG. 7** schematically depicts a closer view of the guidewire 160a and the micro-cannula 150 approaching the niche of the round window 52 (as also depicted in FIGs. 5 and 6). This schematic depiction illustrates that the pathway into the cochlea 50 from the middle ear 40 is a tortuous anatomical pathway. For example, the niche of the round window 52 is typically approached along a first vector 51 which is transverse to the axis of the otic endoscope system 100. Accordingly, as shown, the curve of the guidewire 160a (which induces a similar curve to the micro-cannula 150) can be beneficial to facilitate the advancement of the guidewire 160a and the micro-cannula 150 into the niche of the round window 52 along the first vector 51.

When the guidewire 160a and/or the micro-cannula 150 is/are in the niche of the round window 52, then the membrane of the round window 52 can be punctured. In some embodiments, the guidewire 160a can have a distal tip that can be used for puncturing the membrane of the round window 52. For example, in some embodiments the guidewire 160a can have a sharp distal tip, or other shapes to promote perforation of the round window membrane. In some embodiments, the guidewire 160a is advanced to perforate the round window membrane independently of the micro-cannula 150, or simultaneous with the micro-cannula 150 (with just the sharp tip of the micro-cannula 150 protruding). Perforation of the round window membrane can be done by push advancing the guidewire 160a, or by rotation of the guidewire 160a to "drill" through the round window membrane.

As described elsewhere herein, in some embodiments a second, inner micro-cannula is used inside the main micro-cannula 150 rather than the guidewire 160a. In such a case, the micro-cannula 160a can have a sharp edge at its distal tip, and can be rotated to gently to drill through the round window membrane.

After puncture of the round window membrane, the guidewire 160a (which is also representative of a stylet or inner micro-cannula 160a) can be advanced through the round window membrane. The micro-cannula 150 can be simultaneously or subsequently advanced through the round window membrane over the guidewire 160a. If desired, the guidewire 160a can be exchanged for another guidewire 160a, e.g., if it is desirous to remove the sharp tip or change to a guidewire with different properties (e.g., guidewire stiffness, preformed curvature(s), and the like).

After puncturing the membrane of the round window 52, in order to advance the micro-cannula 150 into the scala tympani 54 of the cochlea 50, the extension and advancement direction of the micro-cannula 150 adjusts from the first vector 51 to approximately align with a second vector 53 extending along the scala tympani 54. Typically, the first vector 51 and the second vector 53 are greatly different from each other (as depicted in FIG. 7). Accordingly, the micro-cannula 150 makes a substantial change in direction (or turn) as it is extended into the scala tympani 54 from its entrance at the round window 52. In some cases, this turn can be executed by first extending the guidewire 160a (with its curved end portion) into the scala tympani 54, and then extending the micro-cannula 150 over the guidewire 160a. In some cases, the guidewire 160a can be withdrawn and a second guidewire 160b (not shown) can be inserted through the micro-cannula 150. The second guidewire 160b can have different properties than the guidewire 160a, such as a curved portion with a different radius than the curved portion of the guidewire 160a.

In some embodiments, after puncturing the membrane of the round window 52, the micro-cannula 150 can be advanced into the scala tympani 54 by itself (without having a guidewire in the end portion of the micro-cannula 150). In such a case, the high compliance or flexibility of the micro-cannula 150 can allow the micro-cannula 150 to readily conform to the anatomy of the scala tympani 54 as the micro-cannula 150 is advanced therein. Moreover, in some embodiments the distal tip of the micro-cannula 150 can be atraumatically configured (e.g., with a donut tip on its distal end) to avoid incurring damage to the wall of the scala tympani 54 as the micro-cannula 150 is advanced therein.

In some embodiments, the micro-cannula 150 can be 39-40 gauge (about a 0.12mm outer diameter). In some embodiments, the outer diameter of the micro-cannula 150 can be between 0.12mm and 0.4mm. In particular embodiments, the micro-cannula 150 can have a graduated gauge (diameter) such that the tip end portion is smaller than more proximal portions of the micro-cannula 150. The diameter graduation(s) of the micro-cannula 150 can be made in increments (step changes) or continuous (gradually changing).

In whichever manner most appropriate, the micro-cannula 150 can be advanced well into the scala tympani 54. Due to the shape of the cochlea 50, during the advancement of the micro-cannula 150 within the scala tympani 54, the micro-cannula 150 changes or turns from extending along the second vector 53 to extending along a third vector 55 as the micro-cannula 150 is advanced (and thereafter extending along other directions defined by the spiral shape of the scala tympani 54). In some embodiments, the micro-cannula 150 can have a lubricious coating to help facilitate its advancement. In some embodiments, the micro-cannula 150 can have a non-circular cross-sectional shape (e.g., D-shaped, ovular, etc.).

The micro-cannula 150 can be advanced into the scala tympani 54 to a desired depth. In some embodiments, depth markers can be included on the micro-cannula 150 so that the clinician can insert the micro-cannula 150 into the scala tympani 54 to a target depth (or a target range of depth) within the cochlea 50. Studies by the inventors have shown that the tip of the micro-cannula 150 need not be positioned all the way to the tip of the cochlea 50. Instead, the inventors have discovered that depth locations well within the speech range, e.g., between the frequency positions of 8000 kHz (about 10mm from the tip of the cochlea 50) and 600 Hz (about 25mm from the tip of the cochlea 50) are good target depths from which to infuse the therapeutic agent. Such a depth is sufficient in part because the inventors have also discovered that the infusion can include a jetting action (or convective flow) by which the therapeutic agent will reach beyond the distal tip of the micro-cannula 150 during the injection. For example, with some permutations (for example, using a flow rate between 1 cc/min and 2.5 cc/min with a 0.13mm x 0.26mm ID x OD micro-cannula), the inventors have achieved convective mixing up to 15mm past the distal tip of the micro-cannula 150 (although the inventors can envision using lower flow rates even down to 5-50 microliter/min, and micro-cannulas sized between 0.12mm - 0.9mm OD).

When the micro-cannula 150 has been inserted into the scala tympani 54 to the target depth, the infusion of the therapeutic agent via the micro-cannula 150 can be started. In some embodiments, the guidewire (if still present within the micro-cannula 150 at this stage) is first removed from the lumen of the micro-cannula 150 prior to the infusion. In particular embodiments, the guidewire (if still present at this stage) is left in the lumen of the micro-cannula 150 during the infusion.

In some embodiments, during the infusion of the therapeutic agent into the cochlea 50 via the micro-cannula 150, natural fluid from the cochlea 50 can be concurrently aspirated. Accordingly, in some embodiments the micro-cannula 150 can be configured with a second lumen that has one or more openings positioned proximally of the distal tip of the micro-cannula 150. In other words, in some embodiments the micro-cannula 150 is a multi-lumen catheter with a first lumen for infusion and a second lumen for aspiration. The one or more openings to the second lumen can be located proximally of the distal tip of the micro-cannula 150 (e.g., near to the round window 52) so that the length of the scala tympani 54 along the catheter is exposed to the infused agent. The two lumens of such a multi-lumen micro-cannula 150 can have a variety of cross-sectional shapes and configurations (e.g., a C-shaped cross-section adjacent to circular cross-section, two circular cross sections, or other shapes). In other embodiments, the multi-lumen micro-cannula 150 can have two nested tubular structures such that the second lumen is concentric with the main lumen. The multi-lumen micro-cannula 150 can, in some embodiments, be comprised of two coaxial tubes such that injection can take place through a center tube and simultaneous aspiration through the annular space around the center tube (e.g., either at the distal opening or through openings in the side of the outer tube that are proximally located in relation to the distal tip of the center tube).

Such aspiration during infusion can help to prevent potentially dangerous elevations of intracochlear pressure. In some embodiments, a pressure measurement of the scala tympani 54 can be taken during the infusion and used to modulate the amount of aspiration to keep the intracochlear pressure within a target range. In some embodiments, such a pressure sensor can be located on the micro-cannula 150 or on a guidewire in the scala tympani 54.

In some embodiments, a fluid balance technique can be used to modulate/control the amount of aspiration during the infusion. That is, the volumetric amount of the aspiration can be measured and controlled to keep it essentially equal to the volumetric amount of the infusion.

Infused fluids can include, but are not limited to, therapeutic agents such as small molecules, large molecule biologics, peptides, oligonucleotides, and gene therapy vectors. Infused fluids can also include tracer molecules to aid in diagnosis of pathology, such as iodine-based agents to enhance x-ray and CT imaging and gadolinium-based contrast agents for enhancement of MRI images.

Systems which allow for aspiration during infusion also allow for safe collection of native perilymph fluid. This fluid bathes the scala tympani and contains molecular markers which can be used to inform disease diagnosis, disease prognosis, disease stage, and response to intervention. Collected fluid can be analyzed for the presence, absence, and relative quantitative expression of a range of molecules including nucleic acids, peptides, proteins, lipids, ions, and other small molecules using established techniques. It is anticipated that the concentration of perilymph in the collected fluid will decrease over time during the procedure as it becomes diluted with the infused agent. A series of collection reservoirs can be put in place to separate the collected fluid by sampling time. Early samples enriched in perilymph can be preferentially assayed for presence of biomarkers to increase potential for detection and quantitation.

Systems which allow for aspiration during infusion also allow for safe collection of the infused agent. Conversely, systems which allow for outflow through a second fenestration in the cochlea or back through the round window result in exposure of the infused agent to the middle ear tissue and Eustachian tube, and result in increased systemic exposure. In the case of gene therapy in particular, limiting exposure outside the cochlea is highly desirable to limit the immune response to the agent.

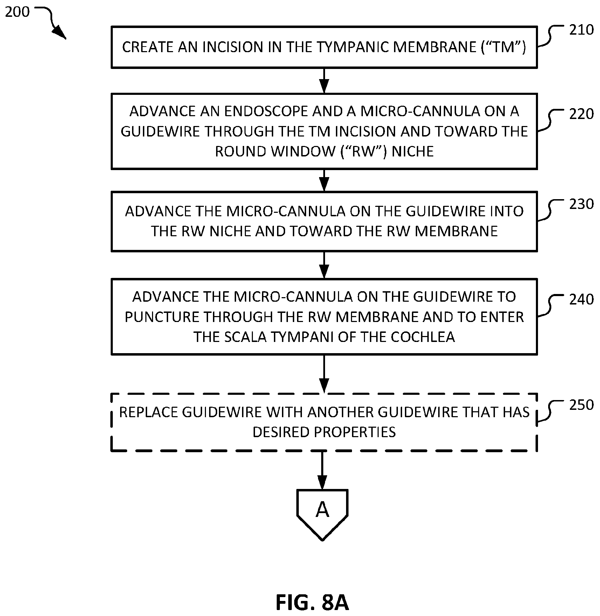

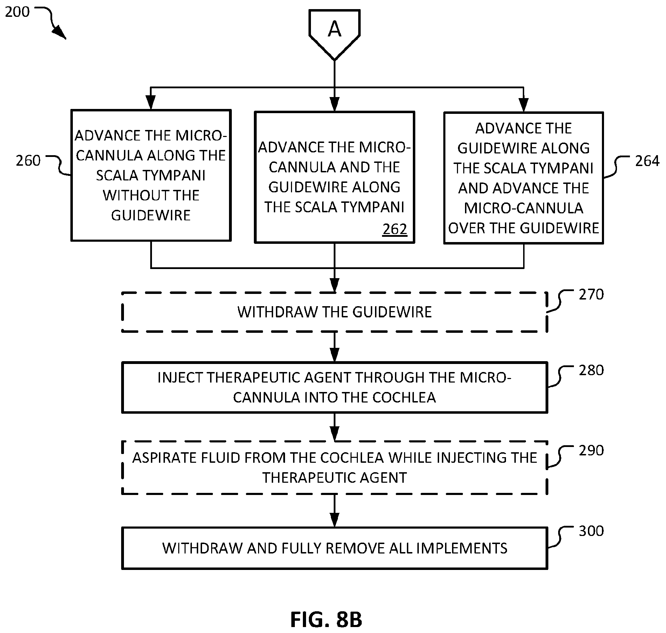

Referring to **FIGs. 8A** **and** **8B****,** a method 200 for infusing a therapeutic substance into a cochlea of a patient is depicted in flowchart format. The method 200 can be performed in the manners described above and using the devices and systems described above. Boxes having dashed lines are optional steps.

In step 210, an incision or opening is created in a tympanic membrane of the patient. Such an incision is exemplified in FIG. 6 (referring to incision 32 in tympanic membrane 30). In some cases, this incision can be less than 2mm in length. In particular situations, a port device can be installed in the incision. Such a port device, residing in the incision, can define an opening through which instruments can extend to access the middle ear. Alternatively, rather than an incision, in some embodiments cases a small tympanic membrane 30 flap can be raised.

In step 220, one or more devices can be advanced through the incision in the tympanic membrane (or opening created by the tympanic membrane flap) and toward the round window niche. For example as depicted and described in reference to FIGs. 5-7, in some embodiments an endoscope, a micro-cannula, and a guidewire can be advanced through the incision in the tympanic membrane. In some embodiments, one or more stabilization devices (e.g., collars, guides, passageways, or tubes that are temporarily anchored to the patient's anatomy and that define one or more channels to slidably receive the instruments), may be used in the outer ear canal and/or at the tympanic membrane. The one or more devices (e.g., endoscope, micro-cannula, and guidewire) can be advanced through such stabilization device(s). The stabilization device(s) can provide enhanced control of the one or more devices and enhanced patient safety. In result, distal end portions of the micro-cannula and the guidewire can be positioned in the middle ear of the patient. The endoscope can be positioned to facilitate visualization of the round window niche, to the extent achievable.

During step 220, in some embodiments the micro-cannula can be located within a working channel of the endoscope and the guidewire can be located within a lumen of the micro-cannula. The micro-cannula can be movable, distally and proximally, in relation to the endoscope. In addition, the guidewire can be movable, distally and proximally, in relation to the micro-cannula. In other words, the micro-cannula is movable over the guidewire.

If the patient has a pseudomembrane or other obstruction to the round window niche, the pseudomembrane or other obstruction can be removed or mitigated to at least the extent necessary to gain access to the round window niche.

In some embodiments, the guidewire can include one or more end portions that are curved or shaped (e.g., as depicted in FIG. 3) to direct the guidewire toward the round window niche. When the micro-cannula is advanced over (or resides on) the curved or shaped portions of the guidewire, then micro-cannula will tend to comply with the guidewire's curved or shaped portions (e.g., as depicted in FIGs. 4 and 7). Accordingly, the guidewire can be used to "steer" the micro-cannula.

In step 230, the micro-cannula is advanced over the guidewire into the round window niche toward the round window membrane that provides a barrier between the round window niche and the scala tympani of the cochlea. The distal tip(s) of the guidewire and/or micro-cannula are in the round window niche at this point.

In step 240, the round window membrane is punctured by the distal tip of the guidewire. In some embodiments, the guidewire may have a tip that is specially constructed for puncturing the round window membrane. For example, in some embodiments the tip of the guidewire can be pointed, sharpened, and/or rigid to facilitate its puncturing of the round window membrane. The micro-cannula can be near to the tip of the guidewire at this junction in order to provide additional column strength to the guidewire to assist with the puncturing of the round window membrane. In some embodiments, the round window membrane can be punctured in other manners (other than by the distal tip of the guidewire). For example, in some embodiments the round window membrane can be punctured using another type of instrument such as a laser, an ultrasonic instrument, and the like.

With the round window membrane punctured, distal tip portions of the guidewire and/or micro-cannula can be advanced through the puncture and into the scala tympani of the cochlea. In some embodiments, a fenestration of the cochlea (cochleostomy) may be created as an alternative entry point to the round window membrane. The cochleostomy can be created via a small drill, a laser, an ultrasonic instrument, and the like. Placement of the micro-cannula through a cochleostomy may facilitate insertion by reducing the degree of navigation required to enter the basal turn of the cochlea.

In optional step 250, the guidewire (or "first guidewire") can be withdrawn and another guidewire (or "second guidewire") that has different properties than the first guidewire can be advanced through the lumen of the micro-cannula (while the micro-cannula remains in its current position). For example, in some embodiments the second guidewire may have a curved portion with a different radius than the first guidewire. In such a case, the second guidewire may facilitate navigation of the tight turn required to advance the micro-cannula from alignment with the first vector 51 to alignment with the second vector 53 (refer to FIG. 7). Moreover, in some embodiments the second guidewire may have an atraumatic tip (e.g., instead of a tip configured for puncturing the round window membrane, as the first guidewire may have). In some embodiments, there can be multiple guidewire change-outs in order to accommodate the multiple turns in the scala tympani.

Steps 260, 262, and 264 are three alternative techniques for advancing the micro-cannula into the scala tympani. In some embodiments, a combination of these three techniques can be used. In particular embodiments, the micro-cannula and/or the guidewire can include depth markers that provide the clinician operator with an indication of the distance to which the micro-cannula and/or the guidewire has/have been extended into the scala tympani.

In step 260, the micro-cannula is advanced along the scala tympani without advancing the guidewire. Said another way, the micro-cannula is advanced along the scala tympani by itself. In some embodiments, the micro-cannula can have a high degree of compliance that allows the micro-cannula to conform to the turns of the scala tympani as the micro-cannula is advanced. In addition, in some embodiments the micro-cannula has an atraumatic tip to mitigate the risk of inflicting damage to the inner wall surfaces of the cochlea as the micro-cannula is advanced.

In step 262 the micro-cannula is advanced along the scala tympani while the guidewire resides in the lumen of the micro-cannula. In other words, the micro-cannula and the guidewire are concurrently advanced along the scala tympani.

In step 264, the guidewire is first advanced along the scala tympani by itself and then the micro-cannula is secondly advanced over the guidewire.

The micro-cannula can be advanced to a particular target depth (or target range of depth) in the cochlea. In general, the tip of the micro-cannula can be inserted to a point that is between the round window and the apex of the cochlea. For example, in some cases the tip of the micro-cannula can be inserted within the cochlea (the scala tympani) from the round window by a distance of between 10mm to 15mm, or 8mm to 20mm, or 12mm to 24mm, or 16mm to 30mm, without limitation.

In step 270, the guidewire within the lumen of the micro-cannula is optionally withdrawn. However, in some embodiments the guidewire is left within the lumen of the micro-cannula.

In step 280, a therapeutic agent is injected or infused into the cochlea through the lumen of the micro-cannula. The infusion can provide a convective jetting effect by which the injectate (therapeutic agent) is injected beyond the tip of the micro-cannula farther toward the apex of the cochlea.

In optional step 290, fluid from within the cochlea can be aspirated while the therapeutic agent is being injected into the cochlea. Such concurrent aspiration during infusion can serve to maintain a fluid pressure in the cochlea within a target range. The aspiration can be active or passive. In some embodiments, a pressure measurement of the scala tympani can be taken during the infusion and used to modulate the amount of aspiration to keep the intracochlear pressure within a target range. In some embodiments, such a pressure sensor can be located on the micro-cannula or on the guidewire in the scala tympani. In some embodiments, a fluid balance technique can be used to modulate/control the amount of aspiration during the infusion. That is, the volumetric amount of the aspiration can be measured and controlled to keep it essentially equal to the volumetric amount of the infusion.

To facilitate the aspiration of step 290, in some embodiments the micro-cannula is a multi-lumen catheter with a first lumen for the infusion and a second lumen for the aspiration. One or more openings to the second lumen can be located proximally (e.g., near to the round window) so that the fluid aspirated from the scala tympani is not the therapeutic agent (or contains only a small amount of the therapeutic agent). In some embodiments, a second cannula can be used to aspirate fluid from the scala tympani near the round window.

In step 300, the implements are withdrawn and fully removed from the patient. The therapeutic agent injected into the cochlea will remain therein so as to provide its therapeutic effects. The small incision to the tympanic membrane 30 can self-heal and close itself over a period of time.

While the method 200 for infusing a therapeutic substance into a cochlea of a patient described above uses an endoscope for direct visualization, in some embodiments a microscope or an OCT (optical coherence tomographic scope) can be used instead of, or in addition to, such an endoscope. Some such cases can include the use of an instrument (or catheter guide) with a small mirror to provide visualization of the round window using the microscope (and the small mirror). Accordingly, in some embodiments no endoscope is needed to perform the method 200.

While the instruments disclosed herein are primarily described in the context of otologic procedures that use a trans-canal, trans-tympanic membrane approach to the middle ear or inner ear, it should be understood that the instruments are not limited to such uses, and could be used for other cavities or spaces in the body and other approaches. For example, in some embodiments the instruments described herein can be used for other approaches and techniques to the middle ear, inner ear, Eustachian tube, mastoid antrum space including, but not limited to, trans-mastoid access, trans-canal via tympanomeatal flap, trans-tympanic membrane annulus, endaural, retroaural, postaural, cochleostomy, and others. Such systems and methods can be used for drug delivery, gel delivery, antibiotic delivery, gene delivery, graft placement, device or implant delivery, tissue removal, diagnostic procedures, sampling procedures, surgical procedures, among others.

It should be noted that any of the embodiments or features of embodiments described herein can be combined in any combinations and any permutations, and all are within the scope of this disclosure.

The devices, systems, and methods described herein may be used in the course of treating any disorder of the middle ear and/or inner ear including, but not limited to, hearing loss, tinnitus, balance disorders including vertigo, Meniere's Disease, vestibular neuronitis, vestibular schwannoma, labyrinthitis, otosclerosis, ossicular chain dislocation, cholesteatoma, otitis media, middle ear infections, and tympanic membrane perforations, to provide a few examples. In some embodiments, the devices, systems, and methods described herein may be used in the course of precise delivery of therapeutic agents to the round window niche and/or other target sites, such as the oval window or other parts of the middle ear cavity, and for providing access to other features or regions of the middle ear. For example, the systems and methods described herein can be used for minimally invasive surgical reconstruction of the ossicular chain, for removal of cholesteatoma, for diagnostic assessment, and other procedures. Any and all such techniques for using the systems and methods described herein are included within the scope of this disclosure.

The devices and systems described herein may be constructed of metals such as but not limited to aluminum, titanium, stainless steel, nitinol, braids of combinations, of these, etc., or of polymers such as but not limited to silicone, polyurethane, ABS, PEEK, PET, HDPE, etc., injection molded components, elastomeric materials, gels, and so on.

The devices, systems, materials, compounds, compositions, articles, and methods described herein may be understood by reference to the above detailed description of specific aspects of the disclosed subject matter. It is to be understood, however, that the aspects described above are not limited to specific devices, systems, methods, or specific agents, as such may vary. It is also to be understood that the terminology used herein is for the purpose of describing particular aspects only and is not intended to be limiting.

A number of embodiments have been described. Nevertheless, it will be understood that various modifications may be made without departing from the claim scope here. Accordingly, other embodiments are possible as long as they are within the scope of the following claims.

## Claims

1. A system (100) for injecting a therapeutic agent into a cochlea (50), the system comprising:
an endoscope having a shaft (120) sized to advance through an outer ear (20) canal and a tympanic membrane (30) such that a distal tip of the endoscope is positionable in a middle ear (40) region for visualization of the cochlea;
the therapeutic agent for delivery into the cochlea;
a micro-cannula (150) including a cochlea delivery lumen, a proximal port in fluid communication with cochlea delivery lumen and configured to receive the therapeutic agent for delivery into the cochlea, and a distal port movable relative to the endoscope to advance adjacent to the shaft of the endoscope and through the tympanic membrane such that the distal port of the micro-cannula is insertable into the cochlea under visualization from the endoscope;
a first guidewire (160a) slidable within the micro-cannula and having a guidewire tip configured to pierce a round window (52) membrane of the cochlea while at least a portion of the first guidewire is positioned within the micro-cannula, wherein the distal port of the micro-cannula is insertable through the round window membrane pierced by the guidewire tip to inject, via the cochlea delivery lumen of the micro-cannula, the therapeutic agent into the cochlea.

2. The system of claim 1, wherein the first guidewire has an end portion with a curved shape, and the micro-cannula is flexible to assume the curved shape of the end portion of the first guide wire in response to advancing the micro-cannula over the end portion of the first guidewire so that the micro-cannula is aligned toward the round window membrane.

3. The system of any of the preceding claims, wherein the distal port of the micro-cannula is insertable through the round window membrane pierced by the guidewire tip and into a scala tympani (54) of the cochlea.

4. The system of claim 3, wherein
the distal port of the micro-cannula is insertable within the scala tympani while the guidewire tip of the first guidewire remains proximal from scala tympani; or
the distal port of the micro-cannula and the guidewire tip of the first guidewire are contemporaneously insertable within the scala tympani; or
the distal port of the micro-cannula is insertable within the scala tympani by advancement of micro-cannula over the first guidewire while the guidewire tip of the first guidewire is positioned within the scala tympani.

5. The system of any of claims 1-3, the first guidewire is withdrawable from the micro-cannula, the system further comprising a second guidewire insertable within the micro-cannula subsequent to withdrawal of the first guidewire from the micro-cannula.

6. The system of claim 5, wherein the second guidewire has a different shape than the first guidewire such that the second guidewire is configured to insert within a scala tympani of the cochlea, and wherein, optionally, the distal port of the micro-cannula is insertable within the scala tympani by advancement of micro-cannula over the second guidewire while the second guidewire is positioned within the scala tympani.

7. The system of any of claims 1-4, wherein the distal port of the micro-cannula is configured to inject, via the cochlea delivery lumen of the micro-cannula, the therapeutic agent into the cochlea while the first guidewire is positioned within the micro-cannula.

8. The system of any of claims 1-4, wherein the distal port of the micro-cannula is configured to inject, via the cochlea delivery lumen of the micro-cannula, the therapeutic agent into the cochlea after withdrawal of the first guidewire from the micro-cannula.

9. The system of any of the preceding claims, wherein the micro-cannula is configured to aspirate fluid from the cochlea during injection of the therapeutic agent into the cochlea.

10. The system of claim 9, wherein
the micro-cannula is configured to aspirate fluid from the cochlea in response to a fluid pressure measured in the cochlea; or
the micro-cannula is configured to aspirate fluid from the cochlea in response to a volumetric balance between the therapeutic agent injected and the fluid aspirated.

11. The system of any of the preceding claims, wherein the micro-cannula includes depth markers positioned proximate to the distal port for visualization from the endoscope, and wherein, optionally, the depth markers are indicative of a depth to which a distal port of the micro-cannula is advanced within the scala tympani.

12. The system of any of the preceding claims, wherein the shaft of the endoscope is sized to advance through an incision (32) in the tympanic membrane that is less than 2mm in length.

## Patentansprüche

1. System (100) zum Injizieren eines Therapeutikums in eine Cochlea (50), wobei das System Folgendes umfasst:
ein Endoskop mit einem Schaft (120), der dazu bemessen ist, durch den Gehörgang (20) und das Trommelfell (30) vorgeschoben zu werden, sodass die distale Spitze des Endoskops im Mittelohrbereich (40) positioniert werden kann, um die Cochlea zu visualisieren;
das Therapeutikum zur Abgabe in die Cochlea;
eine Mikrokanüle (150) mit einem Cochlea-Abgabelumen, einer proximalen Öffnung, die in Fluidverbindung mit dem Cochlea-Abgabelumen steht und dazu konfiguriert ist, das Therapeutikum zur Abgabe in die Cochlea aufzunehmen, sowie einer distalen Öffnung, die relativ zum Endoskop beweglich ist, um neben dem Schaft des Endoskops und durch das Trommelfell vorgeschoben zu werden, sodass die distale Öffnung der Mikrokanüle unter Sichtkontrolle durch das Endoskop in die Cochlea eingeführt werden kann;
einen ersten Führungsdraht (160a), der innerhalb der Mikrokanüle verschiebbar ist und eine Führungsdrahtspitze aufweist, die dazu konfiguriert ist, die Membran des runden Fensters (52) der Cochlea zu durchstechen, während sich mindestens ein Abschnitt des ersten Führungsdrahts innerhalb der Mikrokanüle befindet, wobei die distale Öffnung der Mikrokanüle durch die von der Führungsdrahtspitze durchstochene Membran des runden Fensters eingeführt werden kann, um das Therapeutikum über das Cochlea-Abgabelumen der Mikrokanüle in die Cochlea zu injizieren.

2. System nach Anspruch 1, wobei der erste Führungsdraht einen Endabschnitt mit einer gekrümmten Form aufweist und die Mikrokanüle flexibel ist, um die gekrümmte Form des Endabschnitts des ersten Führungsdrahts anzunehmen, wenn die Mikrokanüle über den Endabschnitt des ersten Führungsdrahts vorgeschoben wird, sodass die Mikrokanüle auf die Membran des runden Fensters ausgerichtet ist.

3. System nach einem der vorstehenden Ansprüche, wobei die distale Öffnung der Mikrokanüle durch die von der Führungsdrahtspitze durchstochene Membran des runden Fensters in die Scala tympani (54) der Cochlea eingeführt werden kann.

4. System nach Anspruch 3, wobei die distale Öffnung der Mikrokanüle in die Scala tympani eingeführt werden kann, während die Führungsdrahtspitze des ersten Führungsdrahts proximal zur Scala tympani verbleibt; oder
die distale Öffnung der Mikrokanüle und die Führungsdrahtspitze des ersten Führungsdrahts gleichzeitig in die Scala tympani eingeführt werden können; oder
die distale Öffnung der Mikrokanüle durch Vorschieben der Mikrokanüle über den ersten Führungsdraht in die Scala tympani eingeführt werden kann, während sich die Führungsdrahtspitze des ersten Führungsdrahts in der Scala tympani befindet.

5. System nach einem der Ansprüche 1-3, wobei der erste Führungsdraht aus der Mikrokanüle herausgezogen werden kann und das System ferner einen zweiten Führungsdraht umfasst, der nach dem Herausziehen des ersten Führungsdrahts aus der Mikrokanüle in diese eingeführt werden kann.

6. System nach Anspruch 5, wobei der zweite Führungsdraht eine andere Form als der erste Führungsdraht aufweist, sodass der zweite Führungsdraht dazu konfiguriert ist, in die Scala tympani der Cochlea eingeführt zu werden, und wobei optional die distale Öffnung der Mikrokanüle durch Vorschieben der Mikrokanüle über den zweiten Führungsdraht in die Scala tympani eingeführt werden kann, während sich der zweite Führungsdraht in der Scala tympani befindet.

7. System nach einem der Ansprüche 1-4, wobei die distale Öffnung der Mikrokanüle dazu konfiguriert ist, das Therapeutikum über das Cochlea-Abgabelumen der Mikrokanüle in die Cochlea zu injizieren, während sich der erste Führungsdraht in der Mikrokanüle befindet.

8. System nach einem der Ansprüche 1-4, wobei die distale Öffnung der Mikrokanüle dazu konfiguriert ist, das Therapeutikum über das Cochlea-Abgabelumen der Mikrokanüle in die Cochlea zu injizieren, nachdem der erste Führungsdraht aus der Mikrokanüle herausgezogen wurde.

9. System nach einem der vorstehenden Ansprüche, wobei die Mikrokanüle dazu konfiguriert ist, während der Injektion des Therapeutikums in die Cochlea Flüssigkeit aus der Cochlea abzusaugen.

10. System nach Anspruch 9, wobei die Mikrokanüle dazu konfiguriert ist, Flüssigkeit aus der Cochlea in Abhängigkeit von einem in der Cochlea gemessenen Flüssigkeitsdruck abzusaugen; oder
die Mikrokanüle dazu konfiguriert ist, Flüssigkeit aus der Cochlea in Abhängigkeit von einer Volumenbilanz zwischen dem injizierten Therapeutikum und der abgesaugten Flüssigkeit abzusaugen.

11. System nach einem der vorstehenden Ansprüche, wobei die Mikrokanüle Tiefenmarkierungen aufweist, die in der Nähe der distalen Öffnung positioniert sind, um vom Endoskop aus sichtbar zu sein, und
wobei die Tiefenmarkierungen optional die Tiefe anzeigen, bis zu der die distale Öffnung der Mikrokanüle in die Scala tympani vorgeschoben wird.

12. System nach einem der vorstehenden Ansprüche, wobei der Schaft des Endoskops dazu bemessen ist, durch einen Einschnitt (32) im Trommelfell mit einer Länge von weniger als 2 mm vorgeschoben zu werden.

## Revendications

1. Système (100) destiné à injecter un agent thérapeutique dans une cochlée (50), le système comprenant :
un endoscope ayant une tige (120) dimensionnée pour avancer à travers un conduit de l'oreille externe (20) et une membrane tympanique (30) de telle sorte qu'une pointe distale de l'endoscope puisse être positionnée dans une région de l'oreille moyenne (40) pour visualiser la cochlée ;
l'agent thérapeutique destiné à être administré dans la cochlée ;
une microcanule (150) incluant une lumière d'administration cochléaire, un orifice proximal en communication fluidique avec la lumière d'administration cochléaire et configuré pour recevoir l'agent thérapeutique destiné à être administré dans la cochlée, et un orifice distal mobile par rapport à l'endoscope pour avancer de manière adjacente à la tige de l'endoscope et à travers la membrane tympanique de telle sorte que l'orifice distal de la microcanule puisse être inséré dans la cochlée sous visualisation à partir de l'endoscope ;
un premier fil-guide (160a) pouvant coulisser à l'intérieur de la microcanule et ayant une pointe de fil-guide configurée pour percer une membrane de fenêtre ronde (52) de la cochlée tandis qu'au moins une partie du premier fil-guide est positionnée à l'intérieur de la microcanule, dans lequel l'orifice distal de la microcanule peut être inséré à travers la membrane de fenêtre ronde percée par la pointe du fil-guide pour injecter, par l'intermédiaire de la lumière d'administration cochléaire de la microcanule, l'agent thérapeutique dans la cochlée.

2. Système selon la revendication 1, dans lequel le premier fil-guide présente une partie d'extrémité ayant une forme incurvée, et la microcanule est flexible de manière à prendre la forme incurvée de la partie d'extrémité du premier fil-guide en réponse à l'avancement de la microcanule sur la partie d'extrémité du premier fil-guide de telle sorte que la microcanule soit alignée en direction de la membrane de fenêtre ronde.

3. Système selon l'une quelconque des revendications précédentes, dans lequel l'orifice distal de la microcanule peut être inséré à travers la membrane de fenêtre ronde percée par la pointe du fil-guide et dans une rampe tympanique (54) de la cochlée.

4. Système selon la revendication 3, dans lequel
l'orifice distal de la microcanule peut être inséré à l'intérieur de la rampe tympanique tandis que la pointe du fil-guide du premier fil-guide reste proximale par rapport à la rampe tympanique ; ou
l'orifice distal de la microcanule et la pointe du fil-guide du premier fil-guide peuvent être insérés simultanément à l'intérieur de la rampe tympanique ; ou
l'orifice distal de la microcanule peut être inséré à l'intérieur de la rampe tympanique par avancement de la microcanule sur le premier fil-guide tandis que la pointe du fil-guide du premier fil-guide est positionnée à l'intérieur de la rampe tympanique.

5. Système selon l'une quelconque des revendications 1 à 3, dans lequel le premier fil-guide peut être retiré de la microcanule, le système comprenant en outre un deuxième fil-guide pouvant être inséré à l'intérieur de la microcanule après le retrait du premier fil-guide de la microcanule.

6. Système selon la revendication 5, dans lequel le deuxième fil-guide présente une forme différente de celle du premier fil-guide de telle sorte que le deuxième fil-guide est configuré pour être inséré à l'intérieur d'une rampe tympanique de la cochlée, et dans lequel, facultativement, l'orifice distal de la microcanule peut être inséré à l'intérieur de la rampe tympanique par avancement de la microcanule sur le deuxième fil-guide tandis que le deuxième fil-guide est positionné à l'intérieur de la rampe tympanique.

7. Système selon l'une quelconque des revendications 1 à 4, dans lequel l'orifice distal de la microcanule est configuré pour injecter, par l'intermédiaire de la lumière d'administration cochléaire de la microcanule, l'agent thérapeutique dans la cochlée tandis que le premier fil-guide est positionné à l'intérieur de la microcanule.

8. Système selon l'une quelconque des revendications 1 à 4, dans lequel l'orifice distal de la microcanule est configuré pour injecter, par l'intermédiaire de la lumière d'administration cochléaire de la microcanule, l'agent thérapeutique dans la cochlée après le retrait du premier fil-guide de la microcanule.

9. Système selon l'une quelconque des revendications précédentes, dans lequel la microcanule est configurée pour aspirer du fluide de la cochlée pendant l'injection de l'agent thérapeutique dans la cochlée.

10. Système selon la revendication 9, dans lequel
la microcanule est configurée pour aspirer du fluide de la cochlée en réponse à une pression de fluide mesurée dans la cochlée ; ou
la microcanule est configurée pour aspirer du fluide de la cochlée en réponse à un équilibre volumétrique entre l'agent thérapeutique injecté et le fluide aspiré.

11. Système selon l'une quelconque des revendications précédentes, dans lequel la microcanule inclut des repères de profondeur positionnés à proximité de l'orifice distal pour être visualisés à partir de l'endoscope, et dans lequel, facultativement, les repères de profondeur indiquent une profondeur jusqu'à laquelle un orifice distal de la microcanule est avancé à l'intérieur de la rampe tympanique.

12. Système selon l'une quelconque des revendications précédentes, dans lequel la tige de l'endoscope est dimensionnée pour avancer à travers une incision (32) dans la membrane tympanique dont la longueur est inférieure à 2 mm.
